(19) Europäisches Patentamt
European Patent Office
Office européen des brevets

(11) **EP 4 137 569 A1**

(12) **EUROPEAN PATENT APPLICATION**
published in accordance with Art. 153(4) EPC

(43) Date of publication:
**22.02.2023 Bulletin 2023/08**

(21) Application number: **21788860.1**

(22) Date of filing: **16.04.2021**

(51) International Patent Classification (IPC):
**C12N 9/38** (2006.01)          **A23L 33/195** (2016.01)

(52) Cooperative Patent Classification (CPC):
**A23L 33/195; C12N 9/2468**

(86) International application number:
**PCT/JP2021/015649**

(87) International publication number:
**WO 2021/210659 (21.10.2021 Gazette 2021/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.04.2020   JP 2020074105**

(71) Applicant: **Godo Shusei Co., Ltd.
Matsudo-shi, Chiba 271-0064 (JP)**

(72) Inventors:
• **HIGA, Yumiko
  Matsudo-shi, Chiba 271-0064 (JP)**
• **OGASAWARA, Junki
  Matsudo-shi, Chiba 271-0064 (JP)**
• **SANO, Ryoko
  Matsudo-shi, Chiba 271-0064 (JP)**
• **BABA, Masahiro
  Matsudo-shi, Chiba 271-0064 (JP)**

(74) Representative: **Hoffmann Eitle
Patent- und Rechtsanwälte PartmbB
Arabellastraße 30
81925 München (DE)**

(54) **PACKAGED LACTASE SOLUTION**

(57)     The purpose of the present invention is to provide a packaged lactase solution that hardly causes clogging of a filter. The packaged lactase solution of the present invention, in which a lactase solution is present in a container, is characterized in that the head space volume in the container is controlled to 20% or less relative to the total volume of the container. Also, the packaged lactase solution is characterized in that the lactase solution has a lactase activity within the range of 10-100,000 NLU/g.

## FIG. 3

HEAD SPACE VOLUME (%)

SLOPE

$y = -0.0036x - 0.1186$
$R^2 = 0.7766$

## EP 4 137 569 A1

**Description**

Field

The present invention relates to a packaged lactase solution that prevents clogging of a filtration filter.

Background

Lactose intolerance refers to a condition in which lactose in foods such as dairy products causes various symptoms such as abdominal pain and diarrhea since a person is congenitally unable to decompose lactose well. Lactose is a disaccharide composed of galactose and glucose. In order to deal with lactose intolerance, lactose contained in milk or the like is preliminarily decomposed into galactose and glucose by the action of lactase in the food manufacturing industry.

The lactase solution used to decompose lactose contained in milk or the like is conventionally obtained by culturing lactase-producing microorganisms, extracting lactase from inside the cells, or acquiring lactase secreted outside the cells, and purifying the lactase by removing foreign substances derived from the culture, then, adding additives such as stabilizers, filtrating and sterilizing the product, and packaging the product in a container for commercialization.

The manufactured packaged lactase solution is stored, sold, and transported under refrigeration (equal to or below 10°C). The lactase solution is then added by the user to milk such as cow's milk or dairy products. There are mainly two methods of adding the lactase solution: one method of adding before sterilization and the other method of adding after sterilization of milk or the like. The former case does not necessarily require a filtration and sterilization step, whereas the latter case requires a filtration and sterilization step for the lactase solution. The milk after addition of the lactase solution is filled before being sold.

In the manufacturing process of adding a lactase solution to milk or the like after sterilization as described above, the lactase solution tends to clog the filter in the filtration and sterilization step, which is known to cause a significant decrease in work efficiency. In order to deal with the problem, for example, Patent Literature 1 (Japanese Examined Patent Application Publication No. 6-73454) describes that the lactase solution is filtrated and sterilized before the formation of degradation products of proteins and polysaccharides that cause clogging, for example, immediately after recovery and purification of the lactase solution. This method can be performed during the production of the lactase solution. However, it cannot be done once the degradation products are formed. This is because clogging may occur in some cases if the lactase solution produced by this method is filtrated after being stored or transported. For example, after transporting the packaged lactase solution produced by this method to a user, if the user performs a filtration and sterilization step for the lactase solution in order to add the lactase solution to milk or the like, clogging may occur in the filter in some cases. When clogging occurs, the manufacturing process must be stopped and the filter must be replaced, which poses a problem of significantly lowering work efficiency. In particular, when the individual manufacturing processes for producing milk are continuous, it is not possible to stop only the filtration and sterilization step, so it becomes necessary to stop all the milk manufacturing processes. As a result, the milk production efficiency is remarkably reduced, thus, an improvement has been desired.

Therefore, the method described in Patent Literature 1 does not solve the problem regarding clogging in filtration of the lactase solution after commercialization.

In addition, Patent Literature 2 (Japanese Translation of PCT International Application Publication (JP-T) No. 2004-534527) describes restricting the concentrations of polysaccharides and oligosaccharides contained in a lactase solution to certain values or less, particularly removing these substances by chromatography. However, even if the concentrations of polysaccharides and oligosaccharides are restricted to certain values or less, the same problem of clogging in filtration after commercialization may occur in some cases as described above.

Patent Literature 3 (International publication WO 2016/060224) is proposed as one that solves the above-mentioned problem.

Citation List

Patent Literature

Patent Literature 1: Japanese Examined Patent Application Publication No. 6-73454
Patent Literature 2: JP-T No. 2004-534527
Patent Literature 3: International Publication WO 2016/060224

Summary

Technical Problem

Among raw materials used in dairy products, a lactase solution is expensive. Dairy producing users dilute the lactase solution, and then, add the diluted solution in-line to the dairy product in some cases, to use the lactase solution without waste. The step of sterilizing the diluted lactase solution with a filtration filter and the step of adding the sterilized lactase solution to the dairy product are continuously performed in-line.

However, when the user dilutes the commercially available lactase solution and adds it to the dairy product as described above, the use of the diluted lactase solution may cause clogging of the filtration filter in the filtration step in some cases. When clogging occurs, the replacement cost of the filtration filter will be incurred, and it will be necessary to stop the entire manufacturing process of dairy products in some cases, so improvements have been desired.

The present invention has an object of providing a packaged lactase solution that hardly causes clogging of a filtration filter.

Solution to Problem

The present invention has solved the problem of the present invention by having the following technical configurations.

(1) A packaged lactase solution in which a lactase solution is present in a container, characterized in that the head space volume in the container is controlled to 20% or less relative to the total volume of the container.
(2) The packaged lactase solution according to (1) above, characterized in that the lactase solution has a lactase activity within the range of 10 to 100,000 NLU/g.
(3) The packaged lactase solution according to (1) or (2) above, wherein the lactase solution is substantially transparent.
(4) The packaged lactase solution according to any one of (1) to (3) above, wherein the material of the container is selected from polyethylene, polypropylene, polystyrene, polyvinyl acetate, polyurethane, polyurethane, polytetrafluoroethylene, and acrylonitrile butadiene styrene resin.
(5) The packaged lactase solution according to any one of (1) to (4) above, wherein the temperature of the lactase solution and the temperature of the container are above 0°C and 20°C or lower.

Advantageous Effects of Invention

According to the present invention, it is possible to provide a packaged lactase solution that hardly causes clogging of a filtration filter.

Brief Description of Drawings

Fig. 1 is a view showing the results of a filter permeability test in (a) Reference Example 1 and (b) Reference Example 2.
Fig. 2 is a view showing the results of a filter permeability test in Example 1.
Fig. 3 is a view showing the relationship between the head space volume and the filter permeability of a packaged lactase solution.
Fig. 4 is a view showing (a) lying position or (b) standing position for packaged lactase.

Description of Embodiments

The present invention is a packaged lactase solution in which a lactase solution is present in a container, having a feature in which the head space volume in the container is controlled to 20v/v% or less relative to the total volume of the container. The head space volume for the packaged lactase solution of the present invention is preferably 15% or less, and more preferably 12% or less, relative to the total volume of the container. The lower limit of the head space volume for the packaged lactase solution of the present invention may be 0% or more, may be more than 0%, may be 1% or more, or may be 2% or more. These upper and lower limits can be combined as appropriate. Hereinafter, the lactase solution present in the container may be referred to as an internal lactase solution in some cases.

It has been found that by setting the content within the above range, the internal lactase solution has excellent filter permeability even when the packaged lactase solution is transported.

This mechanism is considered as follows. Hydrophobic portions of proteins have the property of adhering and binding to each other, and in a normal packaged lactase solution, the high stirring force applied to the internal lactase solution increases the frequency of contact between proteins, hence, the generation amount of protein aggregates increases and the filter permeability of the internal lactase solution deteriorates. In contrast, the packaged lactase solution of the present invention reduces the stirring force applied to the internal lactase solution and reduces the frequency of contact between proteins, thus, formation of protein aggregates is suppressed and the filter permeability of the internal lactase solution can be maintained, by setting the content within the above range.

The head space volume in the container refers to a portion of the total volume of the container, which is occupied by a gas phase. In the packaged lactase solution, the total volume of the container is occupied by the internal lactase solution and the head space volume. The head space volume in the container varies with the amount of the lactase solution to be filled.

The head space volume may be constituted of any gas, such as air, oxygen, nitrogen, rare gas or the like. Air is preferable from the viewpoint of manufacturing cost.

The total volume of the container refers to a portion of the container that can be filled with liquid through the opening of the container. Parts that cannot be filled with liquid through the opening of the container (e.g., hollow parts) are not included.

The method for calculating the total volume of the container is as follows.

(1) Measure the weight of an empty container.
(2) After filling the container with water from the opening of the empty container, measure the weight. Place the container filled with water on a horizontal table and draw a line at the water/head space volume interface (on the container) . As a guideline, the amount of water to be filled is 60 to 80% of the container.
(3) Place the container filled with water in (2) on a horizontal table so that the upper surface and the bottom surface of the container when the line is drawn are reversed. Adjust the amount of water in the container so that the line drawn on the container is aligned with the interface between the water and the head space volume. Measure the weight of the container when the interface is aligned with the line.
(4) Calculate the sum of the value obtained by subtracting the weight of the container (1) from the weight of the water-filled container (2) and the value obtained by subtracting the weight of the container (1) from the weight of the water-filled container (3) . Since the specific gravity of water can be assumed to be 1.00, the sum is the total volume of the container.

The method for calculating the head space volume occupying the packaged lactase solution is as follows.

(1) Calculate the total volume of the container.
(2) Measure the specific gravity of the lactase solution to be filled.
(3) Fill a predetermined weight of the lactase solution into the container through the opening of the empty container.
(4) Divide the predetermined weight of (3) filled by the specific gravity of (2) to calculate the volume of the filled lactase solution.
(5) Calculate the head space volume (%) by applying the values to the following formula.

$$\text{Head space volume } (\%) = 100 - (\text{volume of filled lactase solution in } (4)/\text{total volume of empty container in } (1) \times 100)$$

The method for calculating the head space volume occupying the packaged lactase solution is as described above in detail. As a simple method, after calculating the total volume of the container, a plurality of packaged lactase solutions are prepared in advance so as to have a predetermined head space volume (e.g., 1%, 5%, 10%, 20%, etc.), and the approximate head space volume may be determined by comparing with this.

In the packaged lactase solution, it is preferred to reduce the surface area at the interface between the internal lactase solution and the head space volume. As the surface area increases, bubbles are likely to be generated when the packaged lactase solution is transported, and clogging of the filtration filter tends to be likely to occur.

The activity (or protein concentration) of the internal lactase solution is preferably within the range of 10 to 100,000 NLU/g, more preferably within the range of 100 to 50,000 NLU/g, and further preferably 1,000 to 11,000 NLU/g (FCC4 method). "NLU" is Neutral Lactase Unit. It is preferably within this range both before and after transporting the packaged lactase solution. The lower the activity of the internal lactase solution, the more likely clogging of the filtration filter occurs after transportation of the packaged lactase solution.

The FCC4 method measures the activity by the hydrolysis of a substrate o-nitrophenyl-β-galactopyranoside (ONPG) to o-nitrophenyl and galactose. The reaction is terminated by the addition of sodium carbonate. The o-nitrophenyl formed becomes yellow in an alkaline medium, and the change in absorbance is used to measure the enzymatic activity (expressed in NLU/g). This procedure has been published in U.S. Food Chemicals Codex (FCC), 4th Edition, July 1, 1996, pp. 801-802/lactase (neutral) (β-galactosidase) activity.

The lactase solution of the present invention has an acidic lactase activity of desirably 10 to 100,000 ALU/g, more desirably 100 to 50,000 ALU/g, and further desirably 1,000 to 11,000 ALU/g. "ALU" is Acid Lactase Unit. The activity measurement method is, for example, as follows. It is measured by the hydrolysis of a substrate o-nitrophenyl-β-galactopyranoside (ONPG) to o-nitrophenyl and galactose. The reaction is terminated by the addition of sodium carbonate. The o-nitrophenyl formed becomes yellow in an alkaline medium, and the change in absorbance is used to measure the enzymatic activity (expressed in ALU/g). This procedure has been published in U.S. Food Chemicals Codex (FCC), 4th Edition, July 1, 1996, pp. 802-803/lactase (acidic) (β-galactosidase) activity.

The internal lactase solution of the present invention may be a neutral lactase solution, an acidic lactase solution, or a mixed lactase solution that acts at a neutral to acidic level.

It is preferable that the internal lactase solution is substantially clear. This is because if the internal lactase solution contains a large amount of microorganisms such as lactase-producing bacteria, the internal lactase solution becomes turbid, and the producing bacteria themselves clog the filter. The term substantially clear means that the internal lactase solution is acceptable as long as it is not turbid when visually inspected. The lactase solution may be colored. Specifically, it is a pale yellow to pale brown solution.

The temperature of the packaged lactase solution is preferably above 0°C and 20°C or lower. Aggregates tend to form in the internal lactase solution as the temperature during storage and transportation increases.

The internal lactase solution may contain other proteins besides the lactase protein. Other proteins may be contained in some cases from the viewpoint of the manufacturing cost.

Although protein aggregates are not contained in the internal lactase solution immediately after the production of the packaged lactase solution, the protein aggregates increase as the storage period increases and transportation occurs. The protein aggregates include aggregates between lactase protein molecules, aggregates between lactase protein molecules and other protein molecules, and aggregates between other protein molecules.

Here, in the conventional packaged lactase solution, the container is filled with the lactase solution with a sufficient head space volume for the following two reasons. (1) When the lactase solution is filled by a machine, if the amount of filling is large, the lactase solution may scatter and adhere to the outside of the container, requiring a separate wiping work. In addition, in order to increase the filling amount, it is necessary to lower the filling speed, resulting in complication and lengthening of the manufacturing process. (2) When a user uses a packaged lactase solution with a large filling amount, the lactase solution tends to scatter from the opening of the container, resulting in a problem of handling.

Materials constituting the present invention are described below.

<Container>

The container of the present invention may be one which has an opening through which the lactase solution can be filled into the container, and in which the opening can be sealed. The shape, volume, and material of the container may be appropriately adjusted according to the purpose of use. The container may be sealed, for example, by tightening a screw cap.

The shape of the container is preferably such that it can stand on its own. Plastic containers, drum cans, containers, etc. can be used. Cuboid or rounded corner cuboid plastic containers and containers are preferable because of its excellent loadability.

The volume of the container can be in the range of 10 mL to 20, 000 kL, and preferably in the range of 1 L to 10,000 kL.

As the material of the container, thermoplastic resins such as polyethylene, polypropylene, polystyrene, polyvinyl acetate, polyurethane, polyurethane, polytetrafluoroethylene, acrylonitrile butadiene styrene resin and the like, and metals such as iron, stainless steel and the like can be used in addition to thermosetting resins, and ultraviolet-curable resins. Preferred are thermoplastic resins, and particularly polyethylene and polypropylene. High density polyethylene can be preferably used as the polyethylene.

<<Constituent component of lactase solution>> <Lactase>

(Type of raw material organism)

Lactase has been isolated from a wide variety of organisms including microorganisms. Lactase is often an intracellular or extracellular component of microorganisms such as Kluyveromyces and Bacillus. Kluyveromyces, especially K. fragilis and K. lactis and yeasts such as those of the genera Candida, Torula and Torulopsis are common sources for the yeast enzyme lactase, while B. coagulans or B. circulans is a well-known source for bacterial lactase. Several lactase preparations derived from those organisms are commercially available. All these lactases are so-called neutral lactases since their optimum pH is between pH=6 and pH=8. Aspergillus niger, Aspergillus oryzae, and Penicillium multicolor also produce extracellular lactases, and U.S. Pat. No. 5,736,374 describes examples of such lactases produced by Aspergillus oryzae. The enzymatic properties of lactase, such as optimum pH and optimum temperature, vary from species to species. In general, extracellular lactase is so-called acidic lactase, which has a low optimum pH of from pH=3.5 to pH=5.0.

Additionally, there is also a lactase from Bifidobacterium bifidum that acts at neutral and acidic values (pH 4 to pH 10).

It is also possible to produce the lactase gene derived from these microorganisms by recombination with the host. The host includes, for example, the genera Aspergillus, Kluyveromyces, Trichoderma, Escherichia coli, Pichia, Saccharomyces, Yarrowia, Neurospora, Lactococcus or Bacillus.

In the present invention, it is preferable to use neutral lactase and acidic lactase, and it is particularly preferable to use neutral lactase derived from the genus Kluyveromyces, acidic lactase derived from the genus Aspergillus, and lactase derived from Bifidobacterium.

The lactase solution of the present invention may contain various components as required. Specific examples thereof include metal salts, various sugars, ascorbic acid, glycerin, and the like that contribute to the stabilization of lactase, starch and dextrin which are excipients for improving usability, inorganic salts and the like with a buffering effect, and aggregation inhibitors that make it difficult to generate aggregates in the lactase solution.

(Stabilizer)

The amount of the stabilizer contained in the lactase solution is preferably 10% by mass to 90% by mass, more preferably 20% by mass to 80% by mass, further preferably 30% by mass to 70% by mass, and particularly preferably 40% by mass to 60% by mass. When the amount of the stabilizer is the lower limit or higher, it becomes easier to maintain the lactase activity of the lactase solution over a long period of time. If the amount of the stabilizer exceeds the upper limit, the viscosity of the lactase solution increases, resulting in a longer filtration time and lower workability.

The stabilizer includes, for example, glycerin and sorbitol.

(Aggregation Inhibitor)

The aggregation inhibitors to be contained in the lactase solution include aggregation inhibitors I to III.

The aggregation inhibitor I includes (type 1) HLB 12-15 surfactants, (type 2) lipophilic surfactants, (type 3) nonionic lipophilic surfactants, (type 4) nonionic surfactants, and (type 5) natural product-based surfactants. More preferred are nonionic surfactants with an HLB of 12 to 15. It is understood that the presence of such an aggregation inhibitor in the system reduces or prevents the hydrophobic interaction of proteins, and as a result, the formation of clogging substances due to aggregation can be prevented even when stirring or shaking for a long period of time. Surfactants having an HLB of 12 to 15 are desirable in terms of emulsification stability in an aqueous solution and the high dispersing effect of hydrophobic substances. These types are classified according to physical properties, origin, etc., and a component belonging to one type may belong to another type in some cases. Moreover, multiple surfactants of the same type may be used in combination, or multiple surfactants of different types may be used in combination.

As the aggregation inhibitor II, protective agents having an action of covering the surface of lactase or other proteins can be used. Polyethers and polysaccharide thickeners can be used as the protective agent.

The aggregation inhibitors III include metal ions or salts thereof that have a salt dissolution effect. Among metal ions, a guanidium ion, a calcium ion, a Mg ions, or salts thereof are preferred because they facilitate obtaining an appropriate ionic strength in the lactase solution. The addition of metal ions or salts thereof adjusts the ionic strength of the solution to reduce the interprotein hydrophobic interactions of lactase and other proteins contained in the lactase solution. As a result, it is understood that the protein is less likely to aggregate and has the effect of reducing the formation of clogging substances. Also, a plurality of metal ions of the same type or salts thereof may be used in combination, or a plurality of metal ions of different types or salts thereof may be used in combination.

These aggregation inhibitors I to III may be used alone, or different types of aggregation inhibitors may be used in combination. For example, the aggregation inhibitors I and II, the aggregation inhibitors II and III, the aggregation inhibitors I and III, and the aggregation inhibitors I, II and III may be used in combination. In addition, since the aggregation inhibitor III alone has only a slight effect, it is preferable to use the aggregation inhibitors I and III, and the aggregation inhibitors II and III in combination.

The aggregation inhibitor I can be added to the lactase solution preferably in the range of 0.001% by mass to 5% by mass, more preferably in the range of 0.01% by mass to 1% by mass, and further preferably in the range of 0.1% by mass to 0.5% by mass based on the total mass of the lactase solution.

The aggregation inhibitor II can be added to the lactase solution preferably in the range of 0.05% by mass to 15% by mass, more preferably in the range of 0.3% by mass to 10% by mass, and further preferably in the range of 0.5% by mass to 5% by mass based on the total mass of the lactase solution.

The aggregation inhibitor III has a concentration of metal components in the lactase solution of preferably 0.1 mM or more and 20 mM or less, more preferably 0.25 mM or more and 15 mM or less, further preferably 0.5 mM or more and 10 mM or less, and most preferably 1 mM or more and 5 mM or less. A guanidium ions, a calcium ion, and a magnesium ion are arranged in the descending order of the salt dissolution effect. A magnesium ion requires a higher concentration, whereas a guanidium ion and a calcium ion require lower concentrations.

<<Method for producing lactase solution>>

A method for producing a lactase solution includes, for example, (1) a lactase extraction step involving cell wall destruction after culturing a microorganism such as yeast, and (2) a purification step for removing foreign substances derived from the culture, from the extracted lactase. It may also include (3) a step of adding an additive to the above lactase (which may be prepared immediately before or a commercially available product) as necessary, and (4) a step of filtering for sterilization.

A packaged lactase solution can be obtained by filling a prescribed container with a predetermined amount of the filtrated lactase solution.

<<Method of use/Application of lactase solution>> (Method of using lactase solution)

As a specific utilization form of the lactase solution, for example, it is used in the production of fermented milk. The method for producing lactose-decomposed fermented milk includes: 1. A method of adding lactase to milk before sterilization to decompose lactose and then inactivating the lactase at the same time as sterilizing the milk by heating, to ferment the milk (JP-A No. 5-501197) ; 2. A method of adding lactase to pasteurized milk to decompose lactose, and then inactivating the lactase by a heat treatment, and then fermenting the milk; 3. A method of decomposing lactose in milk with immobilized lactase and then fermenting the milk (JP-A Nos. 46-105593 and 59-162833) ; 4. A method of fermenting a previously lactose-decomposed or lactose-removed raw material using pasteurized milk; etc.

Furthermore, as a specific utilization form of the lactase solution of the present invention, it is used in the production of long-life milk. The long-life milk is long-term storage milk, and the manufacturing process consists of a sterilization step and a continuous aseptic packaging step, and in general, it is processed by an ultra-high temperature short-time sterilization method at 135 to 150°C for several seconds, and filled in a step that enables aseptic packaging of a paper container that has been sterilized with hydrogen peroxide in advance.

The lactase solution added to the long-life milk is generally added after filtration sterilization when filling milk after ultra-high temperature short-time sterilization.

(Application of lactase solution)

The lactase solution according to the present invention is particularly suitable for producing dairy products. Here, the dairy products include milks such as ice cream, long-life milk, and the like; yogurt, fresh cream, sour cream, cheese, and the like. In particular, the lactase solution according to the present invention is suitable for the production of long-life milk.

(Application of lactase and its pH profile)

When considering the application of lactase, it is roughly divided into two types depending on whether it is neutral lactase or acidic lactase. This is dependent on the pH profile in the application. For neutral pH applications, neutral lactases are generally preferred, while acidic lactases may be more suitable for applications in the acidic range.

The present invention will be described below using examples, but the present invention is not limited to these.

Examples

In the following examples, YNL (manufactured by Godo Shusei Co., Ltd., trade name: GODO-YNL2) was used as the lactase solution. GODO-YNL2 is a neutral lactase derived from Kluyveromyces, with an activity of 5,000 NLU/g, a specific gravity of 1.18 (g/mL), and containing 50% (v/v) of glycerol.

(Filter permeability test)

Detailed conditions in this example of the filter permeability test will be described below.

(Procedure for measuring filter permeability)

The following operations were carried out in an environment of 5 to 15°C.

1. The measuring device, the lactase solution sample and distilled water were cooled to the test environment temperature.
2. The lactase solution sample was diluted with distilled water or concentrated by ultrafiltration to adjust the lactase activity to 1,400 to 1,600 NLU/g and mixed well.
3. In the test, a 25 mm stainless steel filter holder (manufactured by PALL, product number 1209 (effective membrane area 3.7 cm$^2$)) was connected to a stainless steel holder with a 47 mm tank (manufactured by Advantech Toyo Co., Ltd., product name "KST-47"), and the connected body was used as a device. The sample permeating part in the filter holder has a constitution having an O-ring, a membrane, a support screen, and an underdrain disk from the entrance side of the measurement sample (in the test of the present invention, the filter of the stainless steel holder with tank and the parts of the filter holding part (support screen and its support) were not attached) . DURAPORE (product name) manufactured by Merck Millipore (pore size 0.22 um, φ25 mm, made of hydrophilic PVDF) was used as the membrane, and Type 316 stainless steel attached to the filter holder was used as the support screen. In addition, in order to adjust the permeation rate, four circular label stickers with a diameter of 0.9 cm (manufactured by A-One Co., Ltd., product name A-one color label 07010) were pasted on the upper part of the support screen (the entrance side of the measurement sample) so as to be bilaterally symmetrical (effective membrane area: 1.26 cm$^2$), and a membrane filter (pore size: 0.22 pm) was set. The membrane was rinsed with a 50% glycerin aqueous solution and attached to a stainless steel filter holder.
4. The lactase solution sample (sample) diluted in 2. was put into the stainless steel holder with tank.
5. Using an air compressor (manufactured by Yaezaki Kuatsu Co., Ltd., product name "KAPSEL-CON YC-3R" or "PC4-15HLM"), a pressure of 0.2 MPa was applied to the stainless steel holder with tank, and an enzyme solution was pumped. The permeated liquid was received in a container such as a beaker, and the amount of permeated liquid was recorded every 10 seconds, and (1) the permeation amount (permeate (kg/m$^2$)) reduced by 1 m$^2$ of the membrane and (2) the permeation rate (flux (kg/min×m$^2$)) were determined by the following method. Further, the slope a of the approximation curve y=ax+b obtained by plotting (1) on the x-axis and (2) on the y-axis was determined.

Calculation formula (conceptual formula)

```
(1) Permeate (kg/m²) = weight (g) of product permeated
at point n/(membrane radius (mm)) × membrane radius (mm) ×
circumference ratio) (m²) × 1000

(2) Flux (kg/min×m²) = (permeate at point n – permeate
at point (n-1))/(permeation time at point n (min) – permeation
time at point (n-1) (min))
```

* n indicates the measurement point. In order to record the permeation amount for every 10 seconds, for example, if the point n is for the permeation amount or permeate at the time of permeation for 10 seconds, then, the point (n-1) is for the permeation amount or permeate at the time of permeation for 0 seconds.

(Reference Example 1)

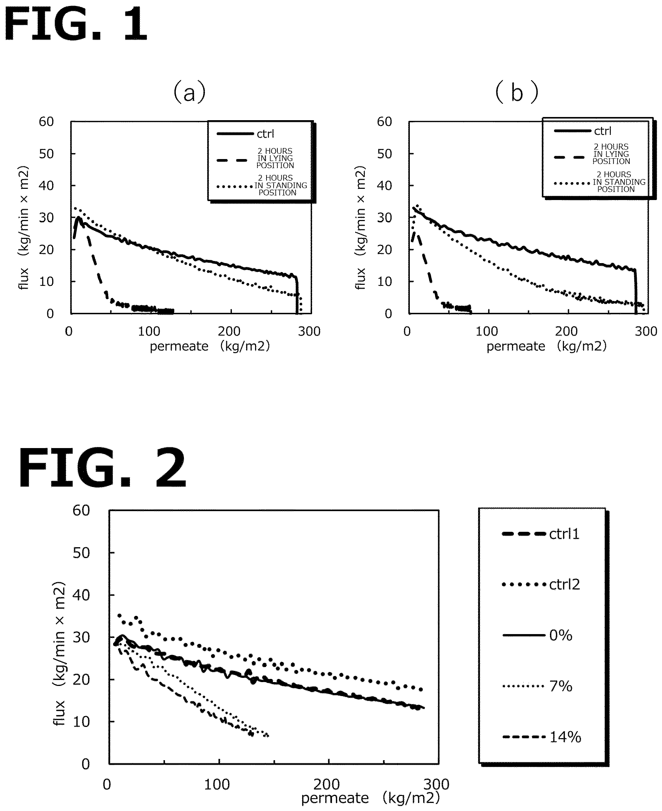

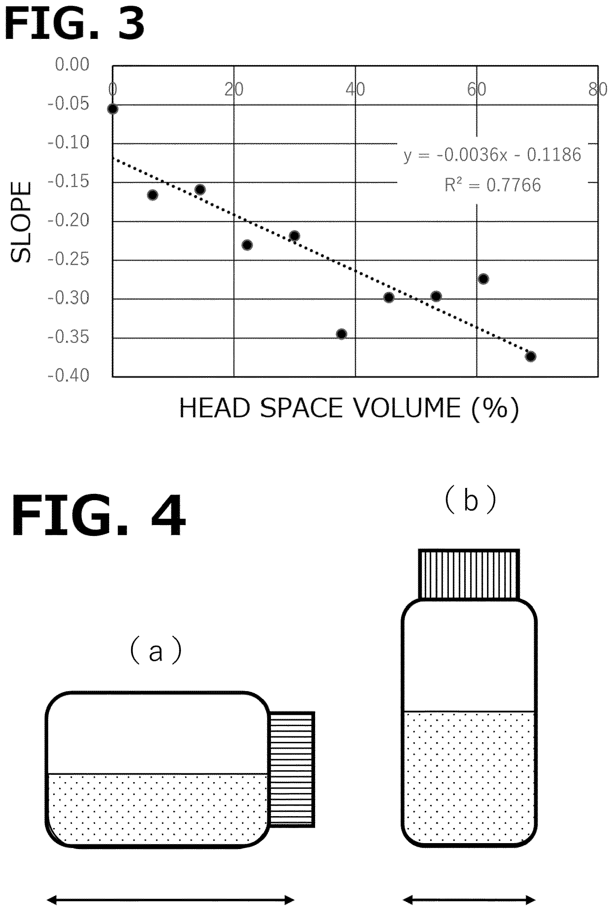

One hundred and seventy grams of a 5,000 NLU/g lactase solution was filled in a 250 mL Iboy (wide-mouthed bottle (product number 5-002-03 manufactured by AS ONE Corporation)) to obtain a packaged lactase solution (60% head space volume). As shown in Fig. 4, the packaged lactase was shaken in (a) lying position or (b) standing position. The shaking conditions were 20°C, amplitude of 30 mm, 100 spm, and 2 hours.
After shaking, the packaged lactase solution was allowed to stand still for 5 hours to conduct a filter permeability test. The one that was not shaken was used as a control. The results are shown in Fig. 1(a). Shaking the packaged lactase solution worsened the filter permeability of the internal lactase solution. The standing position suppressed deterioration of the filter permeability more than the lying position.

This suggests that reducing the surface area of the interface between the internal lactase solution and the head space volume suppresses deterioration of filter permeability.

(Reference Example 2)

In parallel with Reference Example 1, the following test was conducted.

A filter permeability test was conducted in the same manner as in Reference Example 1, except that the packaged lactase solution after shaking was allowed to stand still until the next day. The results are shown in Fig. 1(b). The filter permeability deteriorated depending on the standing time after shaking.

From the results of Reference Examples 1 and 2, it was suggested that when aggregates are formed in the internal lactase solution by shaking the packaged lactase solution, the aggregates grow larger, and the filter permeability deteriorates, as the storage time of the lactase solution increases.

(Example 1)

When the lactase activity value of the internal lactase solution is high, it is difficult to be affected by the filter permeability test by shaking (the internal lactase solution is unlikely to generate aggregates), so the lactase activity value of the internal lactase solution was diluted and tested.

40 mL to 130 mL of a 1,460 NLU/g lactase solution (YNL 2 diluted 3.42 times (weight ratio) with distilled water) was filled in 100 mL Iboy (wide-mouthed bottle (product number 5-002-02 manufactured by AS ONE Corporation), total volume 128.5 mL)) to obtain each packaged lactase solution. Each packaged lactase solution was shaken at 20°C, 100 spm, and amplitude of 30 mm for 1 hour and allowed to stand still at 10°C overnight.

The one that was not shaken was used as a control, and a filter permeability test was performed. Some of the results are shown in Fig. 2. Fig. 2 shows the results after starting the filter permeability test until a permeate of 4.9 to 286.8. However, if the flux is less than 7, it shows the results up to that point. The slope of the graph shown in Fig. 2 was calculated from the linear approximation of Excel and used as an index for the filter permeability test. A larger slope value (negative value) indicates poorer filter permeability. The results obtained are shown in Table 1.

[Table 1]

| Head space volume (%) | Filling amount (mL) | Slope | R2 |
|---|---|---|---|
| ctrl1 | - | -0.0547 | 0.984 |
| ctrl2 | - | -0.0559 | 0.956 |
| 0 | 128.5 | -0.0553 | 0.967 |
| 7 | 120 | -0.1664 | 0.996 |
| 14 | 110 | -0.1593 | 0.977 |
| 22 | 100 | -0.2304 | 0.995 |
| 30 | 90 | -0.2186 | 0.991 |
| 38 | 80 | -0.3450 | 0.991 |
| 46 | 70 | -0.2982 | 0.984 |
| 53 | 60 | -0.2967 | 0.998 |
| 61 | 50 | -0.2742 | 0.991 |
| 69 | 40 | -0.3738 | 0.992 |

The head space volume and slope shown in Table 1 were plotted to create Fig. 3. It was confirmed that the smaller the head space volume of the packaged lactase solution, the more excellent the filter permeability tends to be.

(Example 2) Domestic transportation test of packaged lactase solution

A 10 L plastic container (Tamakan, product number KM-349, manufactured by KODAMA PLASTICS Co., Ltd., total volume 11.7 L in terms of lactase volume) was filled with YNL so that the head space volume was 10 or 28%, to obtain each packaged lactase solution. This packaged lactase solution was transported overland by truck over 22 hours to a location about 600 km away while being stored at 10°C or lower. The packaged lactase solution after transportation was diluted in the same manner as in Example 1 to obtain a 1,460 NLU/g lactase solution, and then a filter permeability test was performed. The results are shown in Table 2. The packaged lactase solution with a filling amount of 10 kg is commercially available from Godo Shusei Co., Ltd. The lots of the internal lactase solution used in Example 2 were the same, and the lots of the internal lactase solutions used in Example 1 and Example 2 were different.

[Table 2]

| Filling amount (kg) | YNL volume (L) | Head space volume (L) | Head space volume (%) | Slope |
|---|---|---|---|---|
| 10 | 8.47 | 3.3 | 28 | -0.04 |
| 12.5 | 10.59 | 1.18 | 10 | -0.024 |

As shown in Table 2, reducing the head space volume of the packaged lactase solution was shown to improve filter permeability. The results shown in Table 2 are for the test under conditions in which aggregated substances were unlikely to occur because the internal lactase solution was transported without being diluted. In the lot used here, one of 10 kg also showed no problem in the filter permeability test. However, since the internal lactase solution is a substance produced by microorganisms, lot differences tend to occur in the filter permeability test. It was shown that it is preferable to decrease the head space volume of the packaged lactase solution to prevent unexpected deterioration of filter permeability.

(Example 3) Overseas transportation test of packaged lactase solution

A 10 L plastic container (Tamakan, product number KM-349, manufactured by KODAMA PLASTICS Co., Ltd., total volume 11.7 L in terms of lactase volume) was filled with YNL so that the head space volume was 1.4 or 56%, to obtain each packaged lactase solution. This packaged lactase solution was transported overland and by sea by truck and ship to a location about 10, 000 km away while being stored at 10°C or lower. The packaged lactase solution after transportation was diluted in the same manner as in Example 1 to obtain a 1, 460 NLU/g lactase solution, and then a filter permeability test was performed. The results are shown in Table 3. The packaged lactase solution with a filling amount of 10 kg is commercially available from Godo Shusei Co., Ltd. The lots of the internal lactase solution used in Example 3 were the same, and the lots of the internal lactase solutions used in Example 1, Example 2 and Example 3 were all different.

[Table 3]

| Filling amount (kg) | YNL volume (L) | Head space volume (L) | Head space volume (%) | Slope |
|---|---|---|---|---|
| 10 | 8.47 | 3.3 | 28 | -0.085 |
| 13.7 | 11.61 | 0.16 | 1.4 | -0.063 |

As shown in Table 3, reducing the head space volume of the packaged lactase solution was shown to improve filter permeability.

**Claims**

1. A packaged lactase solution in which a lactase solution is present in a container, **characterized in that** the head space volume in the container is controlled to 20% or less relative to the total volume of the container.

2. The packaged lactase solution according to claim 1, **characterized in that** the lactase solution has a lactase activity within the range of 10 to 100,000 NLU/g.

3. The packaged lactase solution according to claim 1 or 2, wherein the lactase solution is substantially transparent.

4. The packaged lactase solution according to any one of claims 1 to 3, wherein the material of the container is selected from polyethylene, polypropylene, polystyrene, polyvinyl acetate, polyurethane, polyurethane, polytetrafluoroethylene, and acrylonitrile butadiene styrene resin.

5. The packaged lactase solution according to any one of claims 1 to 4, wherein the temperature of the lactase solution and the temperature of the container are above 0°C and 20°C or lower.

# FIG. 1

(a)

(b)

# FIG. 2

# FIG. 3

$y = -0.0036x - 0.1186$

$R^2 = 0.7766$

HEAD SPACE VOLUME (%)

# FIG. 4

（a）

（b）

**INTERNATIONAL SEARCH REPORT**

International application No.

**PCT/JP2021/015649**

**A.    CLASSIFICATION OF SUBJECT MATTER**

*C12N 9/38*(2006.01)i; *A23L 33/195*(2016.01)i
FI:    C12N9/38; A23L33/195

According to International Patent Classification (IPC) or to both national classification and IPC

**B.    FIELDS SEARCHED**

Minimum documentation searched (classification system followed by classification symbols)

C12N9/38; A23L33/195

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C.    DOCUMENTS CONSIDERED TO BE RELEVANT**

| Category* | Citation of document, with indication, where appropriate, of the relevant passages | Relevant to claim No. |
|---|---|---|
| Y | WO 2016/031885 A1 (GODO SHUSEI CO., LTD.) 03 March 2016 (2016-03-03) paragraphs [0004], [0009]-[0013], [0037], fig. 1, 2 | 1–5 |
| Y | CLARK, J. P., Insights Into Mixing and Blending, Food Technology Magazine, 01 January 2010, vol. 64, no. 1, THE INSTITUTE OF FOOD TECHNOLOGISTS (IFT) [online], <URL:https://www.ift.org/news-and-publications/food-technology-magazine/issues/2010/ january/columns/processing>, [retrieved on 24 June 2021], which retrieved from the internet: <URL:https://www.ift.org/news-and-publications/food-technology-magazine/issues/2010/ january> first paragraph, 'Head Space Requirements' column | 1-5 |
| A | JP 6-073454 B2 (GIST-BROCADES N.V.) 21 September 1994 (1994-09-21) claims 1, 6, page 2, right column, lines 7-50, page 3, right column, lines 12-17 | 1-5 |

☐ Further documents are listed in the continuation of Box C.          ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T"    later document published after the international filing date or priority date and not in conflict with the application but cited to understand the principle or theory underlying the invention

"X"    document of particular relevance; the claimed invention cannot be considered novel or cannot be considered to involve an inventive step when the document is taken alone

"Y"    document of particular relevance; the claimed invention cannot be considered to involve an inventive step when the document is combined with one or more other such documents, such combination being obvious to a person skilled in the art

"&"    document member of the same patent family

| Date of the actual completion of the international search | Date of mailing of the international search report |
|---|---|
| **24 June 2021** | **06 July 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2021/015649**

| Patent document cited in search report | | | Publication date (day/month/year) | Patent family member(s) | Publication date (day/month/year) |
|---|---|---|---|---|---|
| WO | 2016/031885 | A1 | 03 March 2016 | US 2017/0245513 A1<br>paragraphs [0004], [0011]-[0034], [0084]-[0089], fig. 1, 2<br>EP 3187582 A1 | |
| JP | 6-073454 | B2 | 21 September 1994 | EP 145092 A2<br>claims 1, 7, page 2, last line to page 4, line 9, page 6, lines 4-11<br>JP 60-145085 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6073454 B
- JP 2004534527 T
- WO 2016060224 A
- US 5736374 A

- JP 5501197 A
- JP 46105593 A
- JP 59162833 A

**Non-patent literature cited in the description**

- U.S. Food Chemicals Codex (FCC). 01 July 1996, 801-802

- U.S. Food Chemicals Codex (FCC). 01 July 1996, 802-803